# EUROPÄISCHE PATENTSCHRIFT

(11) **EP 2 367 585 B1**
(45) Veröffentlichungstag und Bekanntmachung des Hinweises auf die Patenterteilung: **30.10.2013**
(21) Anmeldenummer: 09759866.8
(22) Anmeldetag: 25.11.2009
(51) Int. Cl.: A61M 15/06

(54) **VORRICHTUNG ZUM ANSAUGEN VON PULVER- UND GRANULAT-MATERIAL UND KAPSEL DAFÜR**
APPARATUS FOR TAKING IN POWDER MATERIAL AND GRANULES AND CAPSULE THEREFOR
DISPOSITIF POUR ASPIRER DU MATÉRIAU EN POUDRE ET EN GRANULÉS ET CAPSULE À CET EFFET

(30) Priorität: 26.11.2008 AT 18472008; 22.12.2008 AT 19982008; 25.06.2009 AT 9872009
(43) Veröffentlichungstag der Anmeldung: 28.09.2011
(73) Patentinhaber: Haas, Rouven, 1140 Wien (AT)
(72) Erfinder: Haas, Rouven, 1140 Wien (AT)
(74) Vertreter: Sonn & Partner Patentanwälte
(86) Internationale Anmeldenummer: PCT/AT2009/000461
(87) Internationale Veröffentlichungsnummer: WO 2010/060127

(56) Entgegenhaltungen:
- EP-A1- 1 867 357
- WO-A1-01/52927
- WO-A1-2007/007110
- WO-A1-2008/052570
- WO-A2-2009/009013
- FR-A1- 2 654 002
- US-A- 4 014 336
- US-A- 4 064 878

## Beschreibung

Die Erfindung betrifft eine Vorrichtung zum Ansaugen von Pulver- oder Granulat-Material aus einer Kapsel, mit einem Saugrohrkörper, der einen Saugkanal und ein Saugende aufweist, und mit einer am Saugrohrkörper dem Saugende gegenüberliegenden Kapsel-Aufnahme und einer Kapselaufstecheinrichtung, gemäß einer Vorrichtung zum Ansaugen von Pulver- oder Granulat-Material aus einer Kapsel, mit einem Saugrohrkörper, der einen Saugkanal und ein Saugende aufweist, und mit einer am Saugrohrkörper dem Saugende gegenüberliegenden Kapsel-Aufnahme und einer Kapselaufstecheinrichtung.

Eine derartige Vorrichtung ist aus der DE 10 2005 043 449 B3 bekannt. Eine ähnliche Vorrichtung ist in der DE 30 16 127 A geoffenbart. Diese bekannten Vorrichtungen sind zum Inhalieren eines Pulvers vorgesehen, das aus einer an beiden Enden perforierten Kapsel freigesetzt wird. Die Vorrichtung weist dabei zwei Gehäuseteile auf, die zusammen eine Kapselaufnahme begrenzen, und von denen der eine einen Saugrohrkörper mit einem Saugkanal ausbildet. Die beiden Gehäuseteile sind mit Kapsel-Aufstechdornen versehen, die z.B. in Form von Pyramiden mit dreieckiger Grundfläche, um eine verbesserte Perforation der Kapseln durch passgenaues Aufschneiden zu erzielen, bzw. in Form von hohlen Nadeln ausgebildet sind. Nachteilig ist jedoch der für diese bekannten Vorrichtungen relativ hohe Aufwand in der Herstellung wie auch in der Handhabung, wobei die zwei Gehäuseteile zum Einfügen einer Kapsel voneinander getrennt und nach dem Einfügen der Kapsel wieder miteinander verbunden werden müssen, wobei die Kapsel an beiden Stirnseiten mit Hilfe der beschriebenen Dorne aufgestochen wird. Der vom Saugrohrköper abgewandte Gehäuseteil sieht dann für das Inhalieren einen Luftansaugteil vor, so dass über diesen Luftansaugteil Luft in und durch die Kapsel gesaugt werden kann, um darin enthaltene Partikel mitzureißen. Bei einer Ausführungsform der Vorrichtung gemäß der DE 30 16 127 A ist nur eine Nadel zum Aufstechen der Kapsel vorgesehen, jedoch ist die Nadel so lange, dass sie die eingelegte Kapsel beim Zusammenschieben der beiden Teleskopteile der Vorrichtung an beiden Enden durchsticht.

Ähnliche Vorrichtungen mit einer langen Nadel oder zwei einander gegenüberliegende Nadeln sind in der WO 2007/106397 A2 gezeigt. Auch hier gilt, dass die jeweilige Kapsel an beiden Enden aufgestochen wird; weiters ist auch hier die Kapselaufnahme innerhalb eines mehrteiligen Gehäuses vorgesehen, d.h. die Kapsel wird immer in einen Hohlraum im Gehäuse eingelegt.

Gemäß der EP 1 867 357 A1 sind mehrere Module, dabei mindestens ein Behältermodul zwischen einem Mundstückmodul und einem Heizmodul, zusammengesteckt; ein Hohlnadel-Kolben wird durch das Mundstückmodul vorgeschoben, um das die Substanz(en) enthaltende Behältermodul an beiden Enden zu perforieren. Weiters ist in einer Ausführungsform ein Behältermodul mit gesonderten Deckeln an beiden Enden vorgesehen, wobei die Deckel einwärts gewölbte Stirnflächen und darin radiale oder kreisförmige Schwächungslinie zum Öffnen der Enden aufweisen.

Aus der US 5,287,850 A ist weiters eine aufwendig ausgebildete Inhaliervorrichtung zur zeitlich gesteuerten und geschwindigkeitskontrollierten Abgabe von Medikamenten in Pulverform geoffenbart, wobei das Innere einer Kapsel mit Überdruck beaufschlagt wird, um diese zum Zerplatzten zu bringen. In diesem Zusammenhang ist auch ein mit einer Aufstechnadel verbundenes Rohr vorgesehen, dem ein Ventil zugeordnet ist.

In der GB 2 253 200 A ist eine zum Einsatz in einer Inhaliervorrichtung vorgesehene aufbrechbare Kapsel gezeigt, wobei das Aufbrechen der Kapsel möglichst leicht erfolgen soll, um das in der Kapsel enthaltene Medikament freizusetzten. Die Kapsel ist dabei verhältnismäßig aufwendig, mit einem Gittereinsatz zum Zurückhalten von Kapselteilen, ausgebildet.

Weiters ist eine aus zwei teleskopartig ineinander gesteckten Teilen bestehende Kapsel aus der DE 198 35 346 A bekannt.

Aus der AT 504 991 A1 ist eine Vorrichtung zur oralen Einnahme von Lebens- oder Genussmitteln beschriebenen, wobei das partikelförmige einzunehmende Material eine bestimmte Maximalgröße bzw. Mindestgröße aufweisen soll, um einerseits die Bildung eines atemwegsgängigen Aerosols zu vermeiden und andererseits zu große Partikel zu vermeiden, die nicht an der Mundschleimhaut anhaften würden. Bei dieser bekannten Vorrichtung ist ein Partikelmaterial-Vorratsbehälter vorgesehen, der Teil der Vorrichtung selbst sein kann, und der wiederum an beiden Seiten Öffnungen aufweist, um das Partikelmaterial aus dem Vorratsbehälter ansaugen und dabei zur Mundschleimhaut lenken zu können. Auch diese bekannte Vorrichtung ist relativ aufwendig in der Ausbildung und vielfach auch nicht besonders einfach in der Handhabung.

In der US 4,014,336 A ist ein Inhaliergerät geoffenbart, das ein allgemein zylindrisches Gehäuse mit einem axialen Luftkanal zwischen einer Eintrittskammer und einer Mundstück-Kammer aufweist; quer zum Luftkanal sind eine diesen schneidende Kapselaufnahmebohrung sowie ein Luftansaugrohr angeordnet, das in der Bohrung ausmündet und vorzugsweise mit einer Schneide an der Mündung versehen ist, um die Stirnseite einer in die Bohrung eingesetzten Kapsel aufzuschneiden. Beim Inhalieren wird Luft durch den Luftkanal und [durch das zum Luftkanal quer verlaufende Luftansaugrohr]angesaugt, wobei Pulver aus der Kapsel mittels Luft, die [durch das zum Luftkanal quer verlaufende Luftansaugrohr] angesaugt wird, freigesetzt und im Luftkanal-Luftstrom mitgerissen wird.

Die WO 01/52927 A1 betrifft ein System zum Inhalieren eines Gases, z.B. Sauerstoff, wobei eine Aluminium-Gaskartusche in ein Gerätegehäuse eingesetzt wird, das ein Ventil mit einem federbeaufschlagten Ventilglied enthält, um beim Ansaugen eine stirnseitige Öffnung der Kartusche freizugeben.

Aus der FR 2 654 002 A1 ist eine Inhaliervorrichtung in Form einer Zigarette oder dergl. bekannt, wobei ein beidseitig mit Öffnungen versehener Behälter für zu inhalierende Substanzen in ein zweiteiliges Gehäuse eingesetzt wird; das Gehäuse weist einen Luftdurchlass auf, in dem überdies Ventile angeordnet sind.

In der WO 2008/052570 A1 ist ein in Form einer Pfeife beschrieben, die geteilt ist und im Bereich der Teilung eine Kammer zur Aufnahme einer Kapsel aufweist, die eine anzusaugende Substanz enthält; überdies ist benachbart der Kapsel eine elektrische Heizeinrichtung vorgesehen.

Die WO 2007/052570 A1 offenbart ein Inhaliergerät mit einem in einem Gehäuse längs verlaufender Luftkanal und einen sich quer dazu erstreckenden Aufnahmeteil für eine Kartusche, in die im Betrieb ein Extraktionsteil mit einer verdickten Spitze hineinragt. Gemäß der US 4,064,878 A wird eine in ein Inhaliergerät eingesetzte Kapsel einseitig mit Hilfe eines Dorns aufgestochen, der zwei Luftkanäle enthält, von denen einer mit der Umgebung zum Zuführen von Luft in die Kapsel und einer mit einen Mundstück zum Ansaugen in Verbindung steht.

Schließlich ist noch auf die aus WO 2009/009013 A2 hergeleitete EP 2 170 444 A2 zu verweisen, die Stand der Technik gemäß Regel 54(3) EPÜ ist und in einer Ausführungsform (gemäß Fig. 22) einen Aufstech-Darm und einen zentralen Luftausgangskanal sowie rund um diese Wirbelluft-Einlasskanäle aufweist; der zentrale Luftansaugkanal endet in einem Querkanal. Die zu inhalierende Substanz ist in einer besonderen Gehäusekonstruktion enthalten, die die Verwirbelung unterstützen soll.

Aufgabe der Erfindung ist die Schaffung einer Vorrichtung wie eingangs angegeben, die eine einfache Herstellung und Handhabung ermöglicht. Insbesondere soll diese Vorrichtung in der Art einer Rauchware verwendbar sein, wobei ähnlich wie bei einer Zigarette oder Zigarre am Saugrohrkörper gesaugt wird, um Partikelmaterial aus einer am Saugrohrkörper angebrachten, an sich üblichen Kapsel anzusaugen.

Zur Lösung der gestellten Aufgabe sieht die Erfindung eine Vorrichtung zum Ansaugen von Pulver- oder Granulatmaterial aus einer Kapsel wie in Anspruch 1 definiert vor; vorteilhafte Ausführungsformen und Weiterbildungen sind in den abhängigen Ansprüchen angegeben.

Bei der Verwendung der vorliegenden Vorrichtung wird die jeweilige Kapsel nur an einer Seite aufgestochen, wobei sie auf der Spitze bzw. auf dem Schaft aufgesteckt bleibt. Die Erfindung macht sich dabei die Erkenntnis zunutze, dass Luft direkt an der Kapselbruchstelle, wo die Spitze in die Kapsel eingedrungen ist, in die Kapsel einströmen kann, wenn am Saugrohrkörper gesaugt wird, wobei die Luft, die in die Kapsel eingeströmt ist, zusammen mit darin verwirbeltem Partikelmaterial aus der Kapsel durch das Loch in den Saugkanal des Saugrohrkörpers einströmt, um dem Mund zugeführt zu werden. Dabei kann im Schaft nur ein radial bzw. quer verlaufendes Loch vorgesehen sein, es können aber selbstverständlich auch mehrere radiale Löcher, z.B. zwei, drei oder vier derartige Löcher vorhanden sein, wobei die Löcher gegebenenfalls einander diametral gegenüber liegen können. Die Spitze kann kegelförmig sein, wobei der Kegel-Scheitelwinkel je nach Material der zu durchstechenden Kapselwand gewählt wird. Beispielsweise beträgt der Scheitelwinkel zwischen 20° und 90°, wobei er umso kleiner ist, je spröder das Material ist, wogegen er umso größer ist, je weicher das Material der Kapselwand ist. Die Kapselwand besteht beispielsweise aus Gelatine oder Zellulosematerial, und Versuche haben gezeigt, dass bei entsprechender Messung immer ein genügend breiter Spalt zwischen dem Schaft der Vorrichtung und dem Rand der in der Kapsel erzeugten Öffnung verbleibt, um dort Luft einströmen zu lassen. Dadurch, dass dabei die Luft in einer Art Gegenstrom in die Kapsel einströmt, wenn am Saugrohrkörper gesaugt wird, kommt es zu einer intensiven Verwirbelung des Partikelmaterials im Kapselinneren, so dass eine sichere Mitnahme dieses Partikelmaterials im Luftstrom zum Mund gegeben ist.

Der Saugrohrkörper samt Schaft und Spitze kann in einem Stück, beispielsweise aus einem Biopolymer, z.B. aus PLA, CAP usw., gefertigt werden. Die Kapsel selbst, die nach dem Aufstechen auf dem Schaft reibschlüssig gehalten wird, kann auch schräg zum Funktionsteil, d.h. zum Saugrohrkörper, angeordnet sein, ohne dass die Funktion beeeinträchtigt würde. Der Kapselinhalt wird bevorzugt derart bemessen, dass das Partikelmaterial bei einem Einsaugen, ungefähr vergleichbar dem Rauchen einer Zigarette, in ca. 5 min bis 10 min oral eingenommen wird. Wenn die Bildung eines Aerosols unerwünscht ist, sollte die Partikelgröße zumindest gleich 30 µm sein, und bevorzugt beträgt sie maximal 150 µm. Das Partikelmaterial kann wasserlöslich sein, mikroskopisch sein und als Basis beispielsweise 60 % Brausepulver aufweisen, so dass eine Aufnahme in der Mundschleimhaut leicht möglich ist. Dem Partikelmaterial kann auch ein Wirkstoff wie etwa Koffein beigemischt sein. Theoretisch ist es aber auch denkbar, die vorliegende Vorrichtung zum Inhalieren eines Aerosol-Medikaments zur Behandlung von Bronchien oder Lungen zu verwenden, wobei dann die Größe des Partikelmaterials z.B. ungefähr 2,5 µm im Mittel betragen könnte.

Um einem etwaigen Ausrieseln des Partikelmaterials nach dem Aufstecken auf die Spitze bei Hochhalten der Vorrichtung entgegenzuwirken, kann vorgesehen werden, dass sich der Schaft in seinem der Spitze benachbarten Teil von der Spitze weg verjüngt, so dass im Bereich des Querlochs eine schräge Fläche vorliegt, die eine Art "Unterschneidung" bildet, so dass in dieser Position kaum Partikelmaterial über das Loch in das Innere des Saugrohrkörpers gelangen kann, wenn nicht gesaugt wird. Außerdem kann zur Vermeidung des Ausrieselns auch vorgesehen werden, mehrere relativ kleine Querlöcher vorzusehen.

Für einen guten Halt der Kapsel am Schaft ist es andererseits zweckmäßig, wenn der Schaft zumindest in seinem Saugrohrkörperseitigen Teil zylindrisch ausgebildet ist. Auf diesem zylindrisch ausgebildeten Teil des Schafts wird die jeweilige Kapsel mit dem Öffnungsbereich, nach Aufstechen bzw. Anbringen der Öffnung in der Kapselwand, reibschlüssig festgehalten, wobei nichtsdestoweniger der Lufteintritt zwischen dem Schaftteil und der Öffnungswand der Kapsel gewährleistet ist.

Um ein zu weites Aufstecken der Kapsel auf die Spitze und den Schaft zu vermeiden, ist es vorteilhaft, wenn der Schaft am von der Spitze abgewandten Ende durch wenigstens einen Kapsel-Anschlagteil begrenzt ist. Der Kapsel-Anschlagteil kann dabei eine gekrümmte Stirnfläche aufweisen, wobei insbesondere eine zylindrische oder auch teilkugelförmige Krümmung vorliegen kann. Bei einer derartigen Ausbildung des Kapsel-Anschlagteils wird auch im Falle eines schrägen Aufsteckens und Aufstechens der Kapsel eine sichere Anlage der Kapsel am Anschlagteil gewährleistet.

Anderseits kann es im Hinblick auf eine besonders einfache Ausbildung auch günstig sein, wenn der Kapsel-Anschlagteil eine plane Stirnfläche aufweist. Dabei ist es weiters für eine einfache Herstellung günstig, wenn der Kapsel-Anschlagteil durch einen die plane Stirnfläche aufweisenden Scheibenteile am Saugrohrkörper gebildet ist.

Es kann aber zur Vermeidung von störend vorstehenden oder vorspringenden Teilen mit Vorteil vorgesehen werden, dass sich der Saugrohrkörper in seinem Querschnitt zum Kapsel-Anschlagteil hin kontinuierlich auf dessen Umriss erweitert.

Für eine mundgerechte Ausführung und angenehme Verwendung kann weiters der Saugrohrkörper am Saugende zu einem Mundstück verdickt sein. Weiters kann, um ein Aufweiten des Saugstrahls unmittelbar vor der Abgabe in den Mund zu erzielen, der Saugkanal zum Saugende hin erweitert sein.

Der Saugrohrkörper kann einen relativ kleinen Querschnitt aufweisen, um bei einem gegebenen Querschnitt des Saugkanals, der sich im Hinblick auf die anzusaugende Partikelmaterial-Menge ergibt, eine nicht zu dicke Wandstärke zu benötigen. Um das Ergreifen der Vorrichtung dabei durch Vergrößerung des Querschnitts zu erleichtern, wird bevorzugt weiters vorgesehen, dem Saugrohrkörper eine über ihn aufschiebbare bzw. aufgeschobene Hülse zuzuordnen. Diese Hülse kann aus Papier oder aus Kunststoff bestehen, wobei eine gewisse Steifigkeit zweckmäßig ist.

Zur Vermeidung eines Ausrieselns von Partikelmaterial durch den Saugrohrkörper, wenn die Vorrichtung gerade nicht in Verwendung ist, kann auch mit Vorteil dem Saugkanal ein beim Ansaugen öffnendes und damit ein den Durchlass des Luftstroms samt Partikelmaterial gestattendes Ventil zugeordnet werden. Dieses Ventil kann im Saugrohrkörper selbst, im Inneren des Saugkanals, vorgesehen werden, es ist jedoch auch möglich, ein derartiges Ventil im Bereich des Quer-Lochs im Schaft der Vorrichtung anzubringen. Dieses Ventil kann dabei zweckmäßig als Membranventil ausgebildet sein, das eine Art Rückschlagventil oder Saugventil (Differenzdruckventil) verwirklicht.

In einer besonders einfachen Ausführungsform der vorliegenden Vorrichtung sind der Schaft und die Spitze koaxial zum Saugrohrkörper angeordnet, d. h. sie haben eine gemeinsame Längsachse, so dass insgesamt ein Zigaretten-ähnliches Aussehen erhalten wird, wenn der Saugrohrkörper geradlinig verläuft.

Es ist jedoch auch günstig, wenn der Schaft und die Spitze mit ihrer Achse winkelig, z.B. rechtwinkelig, zum Saugrohrkörper angeordnet ist. Bei dieser Ausbildung in der Art einer Pfeife, mit hochstehender Kapsel am Saugrohrkörper, kommt in vorteilhafterweise das Partikelmaterial oder Pulver immer am Boden der Kapsel zu liegen, so dass beim Ansaugen immer Partikelmaterial sicher zur Verfügung steht bzw. die maximale Partikelmaterial-Menge im Bereich des Lochs oder der Löcher vorliegt. Dies kann in entsprechender Form auch erreicht werden, wenn der Saugrohrkörper einen in der Art einer Pfeife geschwungenen Verlauf hat. Der Saugrohrkörper kann dabei einfach oder doppelt geschwungen verlaufen, wobei der Schaft mit der Spitze je nachdem entweder in einer Linie mit dem benachbarten Bereich des Saugrohrkörpers oder aber, ähnlich wie bei der vorhergehenden Ausführungsform, z.B. rechtwinkelig hierzu angeordnet sein kann.

Um ein ungewolltes Lösen einer auf die Spitze des Saugrohrkörpers aufgesteckten Kapsel zu verhindern, ist es weiters vom Vorteil, wenn der Schaft auf der dem Saugende näherliegenden Seite des Lochs wenigstens einen Rückhalte-Vorsprung, z.B. in Form eines ringförmigen Wulsts, für eine aufgesteckte Kapsel aufweist. Der Rückhalte-Vorsprung bzw. ringförmige Wulst verhindert hier in der Art eines Widerhakens, dass die aufgesteckte Kapsel von der Spitze des Saugrohrkörpers, d.h. von der Aufstecheinrichtung, abrutschen kann. Die Kapsel liegt dann mit ihren einwärts gebogenen Perforationsrändern am Rückhalte-Vorsprung an und, sofern auch ein Anschlagteil vorgesehen ist, wird sie mit diesem Perforationsrand zweckmäßig zwischen dem Anschlagteil und dem Rückhalte-Vorsprung (Wulst) festgehalten.

Eine weitere vorteilhafte Ausführungsform der vorliegenden Vorrichtung zeichnet sich dadurch aus, dass der Saugrohrkörper im Bereich des Saugendes wenigstens eine Profilierung, z.B. Profilierungen in Form von Rippen oder Nieten aufweist, um das Festhalten des Saugrohrkörpers mit den Lippen bzw. Zähnen zu erleichtern. Die am Saug- oder Mundstückende vorgesehenen Profilierungen, beispielsweise in Form von parallelen Halterippen, ermöglichen ein verbessertes Festhalten des Saugrohrkörpers und damit der Vorrichtung zwischen den Lippen bzw. insbesondere zwischen den Schneidzähnen. Auch können diese Profilierungen bzw. Rippen als dreidimensionale "Markierungen" bzw. als Orientierungshilfe für die Lippen dienen, damit sie und damit der Saugrohrkörper in einer korrekten Ausrichtung zu liegen kommen und festgehalten werden, um die optimale Funktion der Vorrichtung zu gewährleisten. Die Rippen oder Nuten können dabei bogenförmig am Umfang des Saugrohrkörpers vorliegen, sie können auch ringförmig vorgesehen werden, und es können sowohl über die Umfangsfläche des Saugrohrkörpers vorstehende Rippen als auch einfache Vertiefungen in der Umfangsfläche, in der Art von Hohlkehlen vorliegen.

Von Vorteil ist auch eine Vorrichtung mit einer Kapsel mit einem allgemein zylindrischen Kapselkörper, die an den Enden gewölbte Stirnflächen aufweist, wobei - zwecks Erleichtern des bloß einseitigen Aufstechens im Zuge einer händischen Manipulation - an zumindest einer auswärts gewölbten Stirnfläche mittig eine, ein Perforieren mittels der Spitze erleichternde Führungs- oder Schwächungsstelle vorgesehen ist, die durch eine dellenförmige Vertiefung oder eine Materialverdünnung der Kapselwand gebildet ist.

Die Erfindung wird nachfolgend anhand von in der Zeit dargestellten, besonders bevorzugten Ausführungsbeispielen, auf die sie jedoch nicht beschränkt sein soll, noch weiter erläutert. In der Zeichnung zeigen dabei im einzelnen:
Fig. 1 schematisch eine Ansicht einer erfindungsgemäßen Vorrichtung bei der Verwendung, wobei die Vorrichtung teilweise geschnitten veranschaulicht ist;
Fig. 2 den Bereich des Aufstech-Endes dieser Vorrichtung in einem im Vergleich zu Fig. 1 etwas größeren Meßstab;
Fig. 3 diesen vorderen Bereich der Vorrichtung samt aufgesteckter Kapsel bei der Verwendung, wobei die Strömungsrichtungen durch Pfeile veranschaulicht sind;
Fig. 4 eine ganz schematische Schnittdarstellung des vorderen Endes einer solchen Vorrichtung mit einer Modifikation insofern, als eine elastische Membran zur Bildung eines Ventils am Loch im Bereich des Schafts der Vorrichtung vorgesehen ist;
Fig. 5 in den Teilfiguren 5A und 5B schematische Ansichten einer Vorrichtung mit einer gegenüber Fig. 1 modifizierten Ausführung, wobei sich der Saugkanal zum Saugende hin erweitert, u. zw. gemäß Fig. 5A in einer Position unmittelbar vor dem Aufstechen einer Kapsel und in Fig. 5B im Betrieb, bei aufgesteckter Kapsel;
die Figuren 6 und 7 zwei Ausführungsformen von Kapseln mit als Führungs- oder Schwächungsstellen vorgesehenen, das Aufstechen erleichternden dellenförmigen Vertiefungen;
Fig. 8 in den Teilfiguren 8A und 8B in Darstellungen ähnlich jenen von Fig. 5A und 5B eine modifizierte Ausführungsform der erfindungsgemäßen Vorrichtung unmittelbar vor dem Aufstechen einer Kapsel (Fig. 8A) und bei aufgesteckter, aufgestochener Kapsel (Fig. 8B);
Fig. 9 in den Teilfiguren 9A und 9B in vergleichbaren Darstellungen eine weitere modifizierte Ausführungsform der erfindungsgemäßen Vorrichtung vor dem Aufstechen (Fig. 9A) und nach dem Aufstechen (Fig. 9B) einer Kapsel, sowie in den Teilfiguren. 9C und 9D zu den Fig. 9A und 9B gehörige schaubildliche, teilweise aufgeschnittene Darstellungen dieser modifizierten Vorrichtung; und die Teilfigur 9E eine schaubildliche Ansicht der Vorrichtung gemäß Fig. 9A und 9B bei aufgesteckter Kapsel (vgl. insbesondere die Fig. 9B und 9D);
Fig. 10 in den Teilfiguren 10A bis 10E in den Fig. 9A bis 9E vergleichbaren Darstellungen eine weitere Ausführungsform der erfindungsgemäßen Vorrichtung;
Fig. 11 in den Teilfiguren 11A und 11B, in Darstellungen vergleichbar jenen gemäß Figur 5A und 5B, eine weitere, pfeifenartige Ausführungsform der vorliegenden Vorrichtung, wobei in der Darstellung von Fig. 11A die Kapsel gerade aufgesteckt wird und in Fig. 11B der Betrieb durch Luftströmungspfeile angedeutet ist;
Fig. 12 in den Teilfiguren 12A und 12B in ähnlichen Darstellungen wie in Fig. 11A und 11B eine demgegenüber modifizierte Ausführungsform, wobei Lufteintrittsöffnungen im Prinzip ähnlich wie in Fig. 9A bis 9E bzw. 10A bis 10E vorgesehen sind;
Fig. 13 in den Teilfiguren 13A und 13B eine weitere pfeifenartige Ausführungsform der erfindungsgemäßen Vorrichtung, nunmehr jedoch - anders als in Fig. 11A/11B bzw. 12A/12B - mit einem doppelt geschwungenen Saugrohrkörper;
die Figuren 14 und 15 in Teilansichten ähnlich Fig. 2 und 3 eine Ausführungsform der erfindungsgemäßen Vorrichtung im Bereich des Aufstech-Endes, wobei nunmehr in Vergleich zu Fig. 2 bzw. 3 zusätzlich im Bereich des Lochs am Aufstech-Ende ein Rückhalte-Vorsprung für eine aufgesteckte Kapsel vorgesehen ist;
Fig. 16 eine Ausführungsform ähnlich jener gemäß Fig. 8, bei der überdies am Saug- oder Mundstückende des Saugrohrkörpers rippenförmige Profilierungen zum verbesserten Festhalten zwischen den Schneidzähnen vorgesehen sind; und
Fig. 16A eine schematische Detaildarstellung des Saugrohrendes des Saugrohrkörpers von Fig. 16 in vergrößertem Maßstab, zur deutlicheren Veranschaulichung der rippenförmigen Profilierungen.

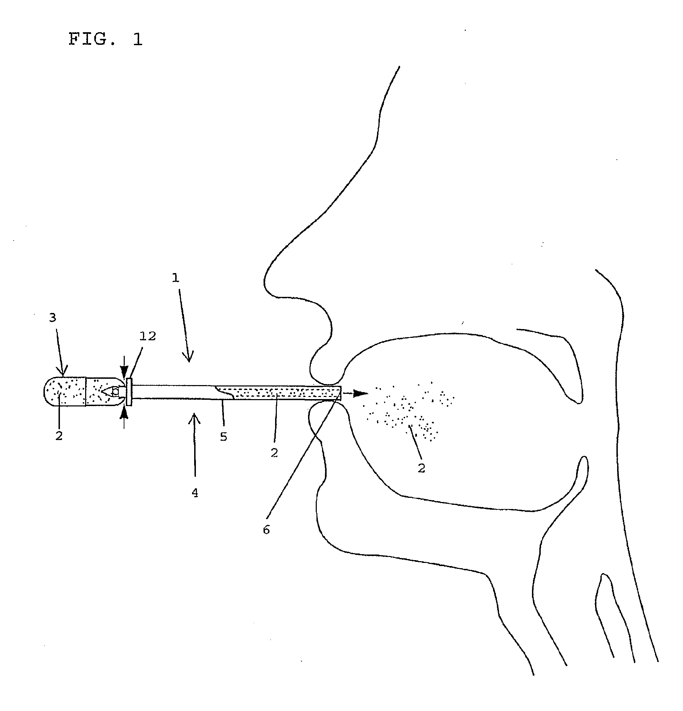

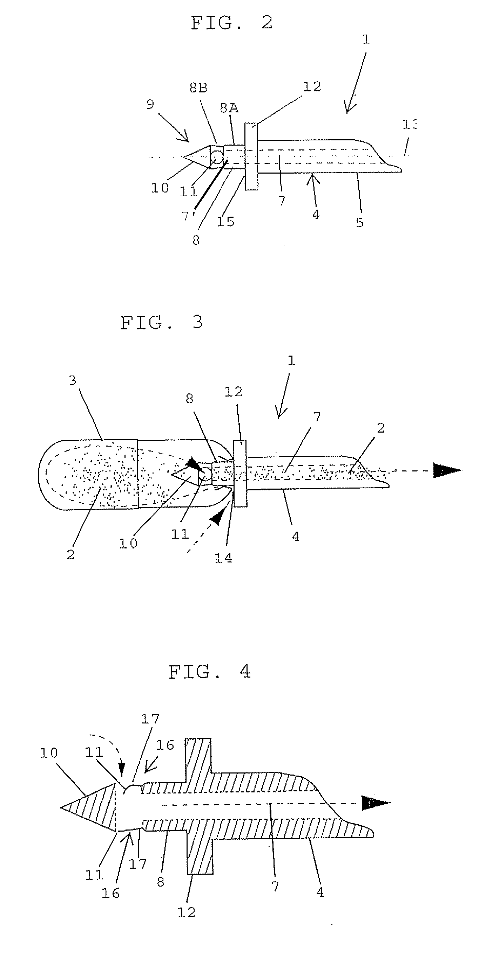

In den Fig. 1 bis 3 ist eine Vorrichtung 1 zum Ansaugen von Pulver- oder Granulat-Material, nachstehend kurz Partikelmaterial 2 genannt, aus einer Kapsel 3 gemäß einer ersten Ausführungsform gezeigt. Die Vorrichtung 1 weist dabei einen Saugrohrkörper 4 auf, der hier in Form eines Hohldorns 5 vorliegt, welcher eine im Wesentlichen zylindrische Gestalt hat und an einem Saugende 6 bei der Benützung der Vorrichtung 1 in den Mund genommen wird, wie in Fig. 1 gezeigt ist. Im Inneren des Saugrohrkörpers 4 bzw. Hohldorns 5 verläuft ein Saugkanal 7, um das Partikelmaterial 2 aus der Kapsel 3 nach deren Aufstechen und Aufstecken auf die Vorrichtung 1, wie in Fig. 1 und 3 gezeigt, ansaugen zu können.

Am dem Saugende 6 gegenüberliegenden Ende des Saugrohrkörpers 4 geht dieser über einen Schaft 8, der einen zylindrischen Teil 8A und einen kegelstumpfförmigen Teil 8B aufweist, in eine als Aufstecheinrichtung 9 vorgesehene kegelförmige Spitze 10 über. Im kegelstumpfförmigen Teil 8B des Schafts 8, der sich von der Spitze 10 weg zum übrigen Saugrohrkörpers 4 hin verjüngt, ist ein radial oder quer verlaufendes Loch 11 vorgesehen, welches mit dem Saugkanal 7 des Saugrohrkörpers 4 über einen Saug-Längskanal 7' (Fig. 2) in Verbindung steht.

Der Schaft 8 wird durch einen Anschlagteil 12 nach hinten, d.h. zum Saugrohrkörper 4 hin, begrenzt, wobei dieser Anschlagteil 12 als Anschlag für das Aufstecken der Kapsel 3 dient, s. Fig. 1 und 3.

Der Saugrohrkörper 4 bzw. Hohldorn 5 erstreckt sich bei diesem Ausführungsbeispiel gemäß einer gemeinsamen Längsachse 13, die mit einer strichpunktierten Linie in Fig. 2 veranschaulicht ist, und die zugleich die (Längs-)Achse des Schafts 8 und der Spitze 10 ist.

Wie insbesondere aus Fig. 1 und 3 ersichtlich ist, wird bei der Verwendung der Vorrichtung 1 eine jeweilige Kapsel 3 nur einseitig aufgestochen, indem sie auf die Spitze 10 und den Schaft 8 aufgesteckt wird, bis sie am Anschlagteil 12 zur Anlage kommt. Dabei wird eine Öffnung 14 am einen Stirnende der Kapsel 3 aufgestochen oder aufgeschnitten, wobei sich die Öffnungsränder einwärts umbiegen können, wie in Fig. 3 veranschaulicht ist. Wie weiters mit Pfeilen in Fig. 1 und 3 dargestellt ist, kann Luft einerseits durch diese Öffnung 14, in Form eines Spalts zwischen dem Öffnungsrand der Kapsel 3 und dem Schaft 8, in einer beschränkten Durchflussmenge einströmen, wobei sie im Inneren der Kapsel 3 verwirbelt wird und dabei das Partikelmaterial 2 mitverwirbelt. Der Strom aus eingedrungener Luft und Partikelmaterial tritt dann, beim Ansaugen am Saugende 6 (Fig. 1), durch das Loch 11 in den Saugkanal 7 ein, wodurch das Partikelmaterial 2 gemäß Fig. 1 in die Mundhöhle transportiert und dort beispielsweise über die Mundschleimhaut aufgenommen wird.

Durch die "Verschrägung" des Teils 8B mit dem Quer-Loch 11 in der Art einer Hinterschneidung wird einem Ausrieseln des Partikelmaterials 2 bei einem Hochhalten der Kapsel 3 nach oben entgegen gewirkt.

Die Spitze 10 hat einen auf das Material der Kapseln abgestimmten Kegel-Scheitelwinkel, wobei sie umso spitzwinkeliger ist, je spröder das Material ist, bzw. umso kürzer sein kann, je weicher das Material der Kapselwand ist. Die aufgesteckte Kapsel 3 wird reibungsschlüssig am Schaft 8 gehalten, der deshalb in seinem Teil 8A (s. Fig. 2) benachbart dem eigentlichen Saugrohrkörper 4 bevorzugt zylindrisch ausgebildet ist, wobei die Bruchkante der Öffnung 14 den Schaft 8 abschließt, ohne das der Eintritt von Luft komplett gesperrt würde. Der Anschlagteil 12 verhindert, dass die Kapsel 3 zu weit auf die Vorrichtung 1 aufgeschoben wird. In der Ausführungsform gemäß Fig. 1 bis 3 hat dieser Anschlagteil 12 eine plane Stirnfläche 15 für die Anlage der Kapsel 3, wobei der Anschlagteil 12 in Form einer zylindrischen Scheibe ausgebildet ist.

In der Ausführungsform gemäß Fig. 4 - die im Wesentlichen jener gemäß Fig. 1 bis 3 entspricht - ist im Bereich des quer verlaufenden Sauglochs 11 als Ventil 16 eine elastische Membran 17 angebracht, die als Saugventil (Rückschlag- oder Differenzdruckventil) wirkt, um beim Saugen durch den Saugkanal 7 das Loch 11 freizugeben, um so den angesaugten Luftstrom mit dem Partikelmaterial gemäß dem in Fig. 4 strichliert eingezeichneten Pfeil eindringen zu lassen. Aus Fig. 4 ist weiters ersichtlich, dass auch mehrere Quer-Löcher 11, z.B. zwei einander diametral gegenüberliegende Löcher 11, vorgesehen sein können. Auch Ausführungen mit drei oder vier Löchern 11 sind selbstverständlich denkbar. Bei der Darstellung in Fig. 4 ist die Membran 17 bzw. das Ventil 16 an der Unterseite der Vorrichtung in der Schließstellung gezeigt, wogegen die Membran 17 an der Oberseite in der Offenstellung veranschaulicht ist. Durch das Ventil 16 bzw. die Membran 17 wird ein Ausrieseln des Partikelmaterials bei aufgesteckter Kapsel verhindert. Bei Einsetzen der Sogströmung öffnet sich jedoch das Ventil 16, d.h. die elastische Membran 17, nach innen und ermöglicht so die angestrebte Funktion des Transports des Partikelmaterials im Luftstromzum Mund des Benützers hin.

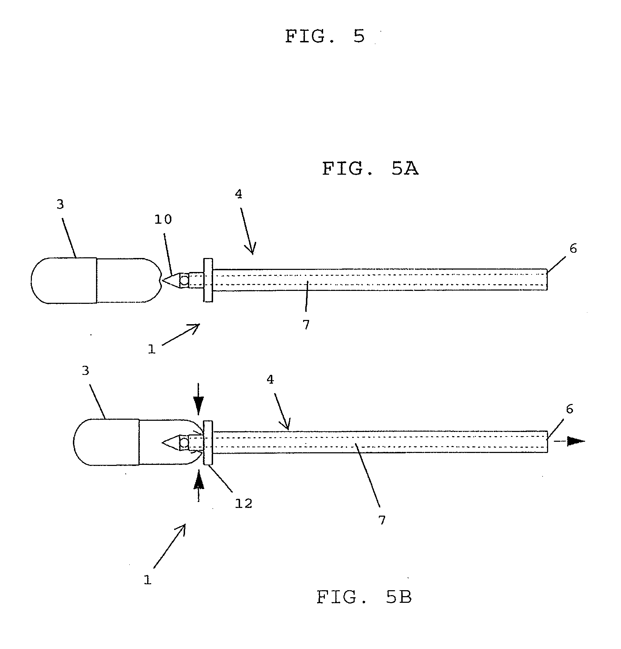

Die Ausführungsform der Vorrichtung 1 gemäß Fig. 5A und 5B entspricht im Wesentlichen jener gemäß Fig. 1 bis 3, wobei als Unterschied nur vorgesehen ist, dass sich der Saugkanal 7 im Inneren des Saugrohrkörpers 4 zum Saugende 6 hin kontinuierlich erweitert. Dadurch wird auch der Partikelmaterial-Strahl beim Eintritt in die Mundhöhle aufgeweitet, um so das Partikelmaterial gut über die Mundhöhle zu verteilen.

Im Übrigen entspricht die Ausführungsform gemäß Fig. 5A und 5B jener gemäß Fig. 1 bis 3, so dass sich eine neuerliche detaillierte Bschreibung erübrigen kann.

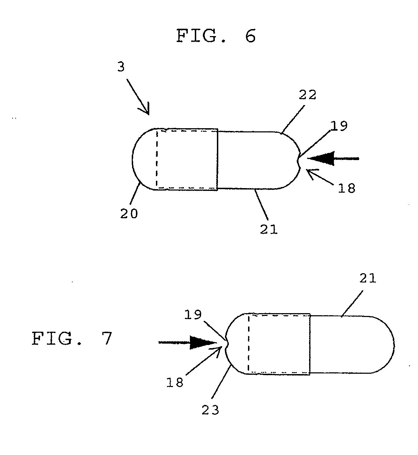

In den Fig. 6 und 7 sind zwei Ausführungsbeispiele für eine Kapsel 3 gezeigt, die mit einer Führungs- bzw. Schwächungsstelle 18 in Form einer dellenförmigen Vertiefung 19 versehen sind, um das Aufstechen zu erleichtern. In beiden Fällen besteht die Kapsel 3 aus zwei Kapselhälften 20, 21, wie dies an sich bekannt ist, wobei diese beiden Kapselhälften 20, 21 nach Befüllen mit dem in Fig. 6 und 7 nicht ersichtlichen Partikelmaterial 2 ineinander gesteckt werden, wobei entweder ein strenger Passsitz gegeben ist oder eine nachträgliche klebende Verbindung vorgesehen wird. Im Fall der Fig. 6 befindet sich die Vertiefung 19 an der teilsphärischen Stirnseite 22 der einen kleineren Querschnitt aufweisenden Kapselhälfte 21, wogegen beim Ausführungsbeispiel gemäß Fig. 7 die eine Kappe bildende größere Kapselhäfte 20 an ihrer gewölbten Stirnseite 23 mit der Vertiefung 19 versehen ist.

Auf diese Weise kann, wie schematisch in Fig. 6 und 7 mit einem Pfeil angedeutet ist, die jeweilige Kapsel 3 punktgenau mit der Spitze 10 der Vorrichtung 1 angestochen werden, wobei wie erwähnt ein einseitiges Anstechen genügt, um die Kapsel 3 mit Hilfe der vorliegenden Vorrichtung 1 durch ein dosiertes Saugen sukzessive zu entleeren.

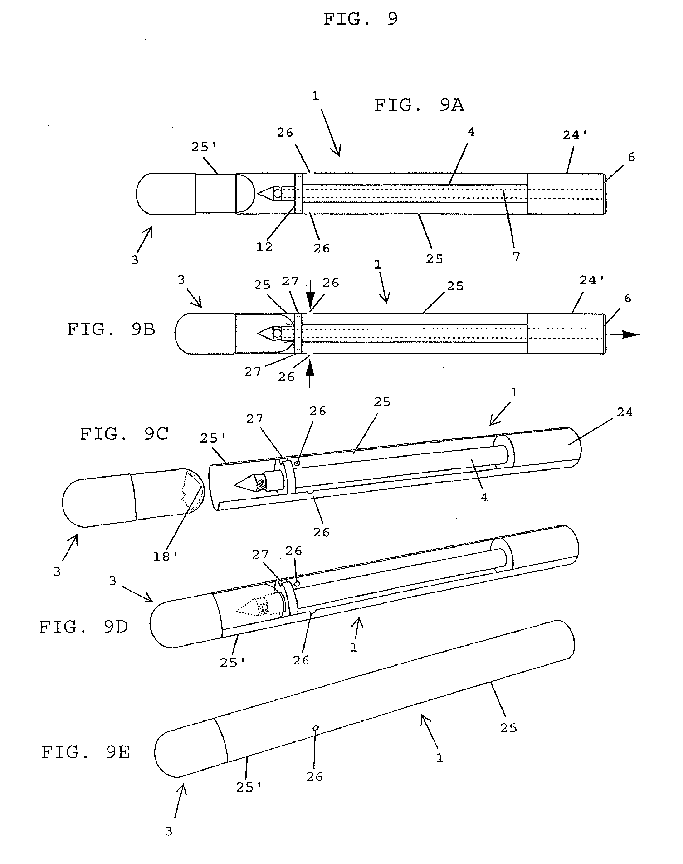

Die Vertiefung als Führungsmittel oder auch Schwächungsmittel kann zu einer Verdünnungsstelle führen, d.h. zu einer Materialverdünnung der Kapselwand an der Stelle 18. Denkbar ist es auch, am Scheitel der jeweiligen gewölbten Stirnwand 22 oder 23 einfach eine Materialverdünnung ohne Führungs-Vertiefung vorzusehen, wie dies in Fig. 9C schematisch bei 18' gezeigt ist.

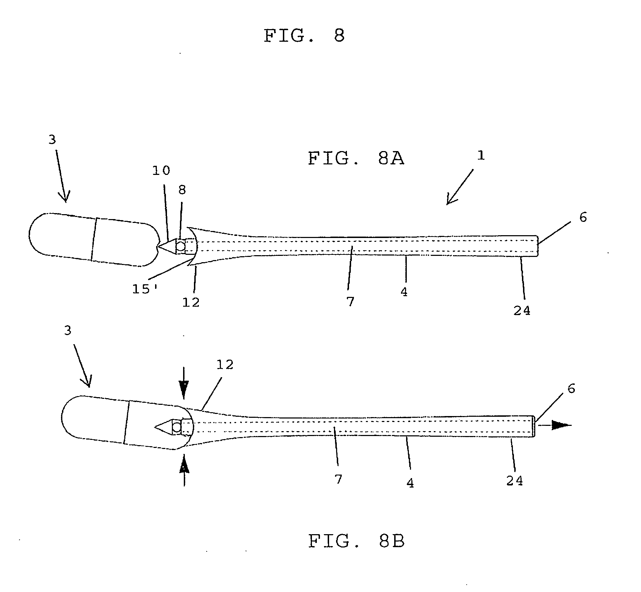

In Fig. 8A und 8B ist eine weitere Modifikation der vorliegenden Vorrichtung 1 zum Ansaugen von Partikelmaterial aus einer auf die Spitze 10 und den Schaft 8 aufgesteckten Kapsel 3 gezeigt, wobei wiederum in Fig. 8A die Phase des Aufstechens der Kapsel 3 und des Aufsteckens derselben und in Fig. 8B die bereits voll aufgesteckte Kapsel 3 gezeigt ist. Die Spitze 10 und der Schaft 8 entsprechen wiederum in ihrer Ausbildung jener gemäß Fig. 1 bis 3 oder Fig. 5A und 5B, so dass sich eine neuerliche Erläuterung erübrigen kann. Weiters ist in der Ausführungsform gemäß Fig. 8A, 8B ähnlich wie bei jener gemäß Fig. 5A, 5B der Saugkanal 7 zum Saugende 6 hin erweitert. Gemäß Fig. 8A und 8B erweitert sich dabei in entsprechender Weise der Außendurchmesser des Saugrohrkörpers 4 zum Saugende 6 hin, so dass eine sich vom hinteren Saugende 6 zur vorderen Spitze hin leicht verjüngende Außenform des Saugrohrkörpers 4 erhalten wird. Der Saugrohrkörper 4 erweitert sich jedoch im letzten Bereich vor dem Schaft 8 und der Spitze 10 (beispielsweise konisch) zum Anschlagteil 12 für die Kapsel 3. Weiters ist hier als Stirnfläche des Anschlagteils 12 eine gekrümmte Stirnfläche 15', im Gegensatz zur ebenen Stirnfläche 15 beim Anschlagteil 12 gemäß Fig. 1 bis Fig. 3, vorgesehen. Diese gekrümmte Stirnfläche 15' kann beispielsweise teilsphärisch oder aber auch teilzylindrisch gekrümmt sein. Auf diese Weise kann die Kapsel 3 außer koaxial zum Saugrohrkörper 4 auch relativ zu letzterem schräg aufgesteckt werden, wie aus Fig. 8A und insbesondere aus Fig. 8B ersichtlich ist, wobei nichtsdestoweniger eine gute Anlage an der Stirnfläche 15' des Anschlagteils 12 gesichert ist. Diese Ausführungsform eignet sich insbesondere für ein ungeführtes Aufstecken der Kapsel 3, wenngleich die Kapsel 3 selbstverständlich auch hier eine Vertiefung 19 oder dergleichen Führungs-/Schwächungsstelle 18 (s. Fig. 6 und 7) aufweisen kann.

Auch hier kann die Vorrichtung 1, d.h. der Saugrohrkörper 4 samt Anschlagteil 12, Schaft 8 und Spitze 10, einstückig gefertigt sein. Durch die Verdickung des Saugrohrkörpers 4 zum Saugende 6 hin wird an diesem Saugende 6 ein Mundstück 24 erhalten. Weiters wird durch die Erweiterung des Saugkanals 7 zum Saugende 6 hin ein Verkleben des angesaugten Materials am Saugende 6 durch einfließenden Speichel minimiert.

Bei der Ausführungsform der Vorrichtung 1 gemäß den Fig. 9A bis 9E ist zusätzlich zum Saugrohrkörper 4 - der beispielsweise in der Form wie in Fig. 1 bis 3 (oder in Fig. 5A und 5B oder aber Fig. 8A, 8B) gezeigt, ausgebildet sein kann - eine äußere Hülse oder Hülle 25 vorgesehen, die am mundseitigen Ende des Saugrohrkörpers 4 - der dort zu einem Mundstück 24 verdickt ist - in einem Mundstückbereich 24' reibschlüssig aufliegt und an der Vorderseite am Anschlagteil 12 außen satt anliegt. Diese Hülse 25 erstreckt sich über diesen Anschlagteil 12 nach vorne hinaus, so dass sie eine Führung 25' für die jeweilige Kapsel 3 bei deren Aufstechen und Aufstecken bildet, wie insbesondere aus den Fig. 9A und 9B bzw. 9C und 9D erkennbar ist. Die Hülse 25, die in den Fig. 9C und 9D aufgeschnitten gezeigt ist, weist beispielsweise zwei Lufteinlässe 26 unmittelbar hinter dem Anschlagteil 12 auf, und benachbart diesen Lufteinlässen 26 weist der Anschlagteil 12 an seinem Außenumfang z.B. achsparalelle Luftkanäle 27 auf, um so dem Aufsteckbereich der Kapsel 3 von der Umgebung her Luft zuströmen zu lassen, wenn am Mundstück 24 bzw. 24' gesaugt wird. Diese über die Lufteinlässe 26 und Luftkanäle 27 einströmende Luft gelangt wiederum in den Spalt zwischen dem Schaft 8 und dem Rand der aufgestochenen Öffnung der Kapsel 3 in das Kapselinnere, um dort das Partikelmaterial zu verwirbeln und das Ansaugen desselben durch den Saugkanal 7 zu ermöglichen, wie dies vorstehend anhand insbesondere der Fig. 1 bis 3 erläutert wurde.

Dadurch, dass die Hülse 25 in ihrem vorderen Bereich eine Führung 25' für die Kapsel 3 bei deren Aufstecken bildet, ist hier eine spezielle Führungs- bzw. Schwächungsstelle 18 an der Kapsel 3 nicht erforderlich. Gegebenenfalls kann jedoch, um das Aufstechen selbst zu erleichtern, wie erwähnt eine Materialverdünnung 18' an einer mittigen Stelle an der Stirnseite der Kapsel 3 vorgesehen sein, vgl. Fig. 9C.

Die Hülse oder Außenhülle 25 kann aus einem ganz einfachen Material, wie z.B. aus Papier, bestehen. Ein besonderer Vorteil dieser Ausführungsform ist, dass durch den Durchlassquerschnitt der Lufteinlässe 26 und der Luftkanäle 27 der Saugwiderstand gesteuert werden kann, um so zusätzlich eine Dosierungshilfe beim Ansaugen von Partikelmaterial 2 aus dem Inneren der Kapsel 3 zu ermöglichen.

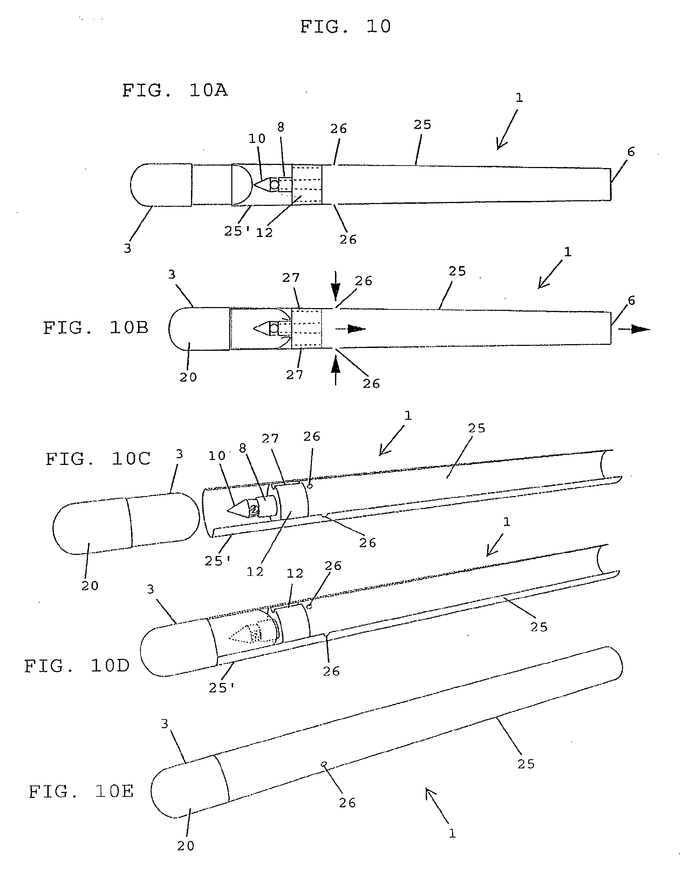

In den Fig. 10A bis 10E ist in den Darstellungen von Fig. 9A bis 9E vergleichbaren Darstellungen eine demgegenüber weiter modifizierte Ausführungsform gezeigt, wobei nunmehr die Hülle 25 selbst den Saugrohrkörper bildet und im Inneren der Hülle 25 als Funktionsteil nur ein Kapsel-Anschlagteil 12 samt Schaft 8 und Spitze 10 vorhanden ist. Bei dieser Ausführungsform wird die Hülle 25 aus einem relativ formstabilen, tragenden Kunststoff, etwa ähnlich einem Trinkhalm gefertigt, und sie kann sich, abgesehen von einer möglichen zylindrischen Form, ähnlich wie gemäß Fig. 9A bis 9E, auch konisch zum Saugende 6 hin verjüngen. Nichtsdestoweniger sollte der vordere Führungsteil 25' für die Kapsel 3 bei deren Aufstechen und Aufstecken auf den Schaft zylindrisch sein. Die Stirnseite der Hülse 25 kann hier ebenso wie im Fall der Ausführungsform gemäß Fig. 9A bis 9E bei aufgesteckter Kapsel 3 an der Grenzfläche zwischen den beiden Kapselhälften, und zwar an der Stirnseite der äußeren, kappenartigen Kapselhälfte 20, zu liegen kommen, wie aus den Fig. 10B, 10D und 10E (bzw. 9B, 9D, 9E) ersichtlich ist.

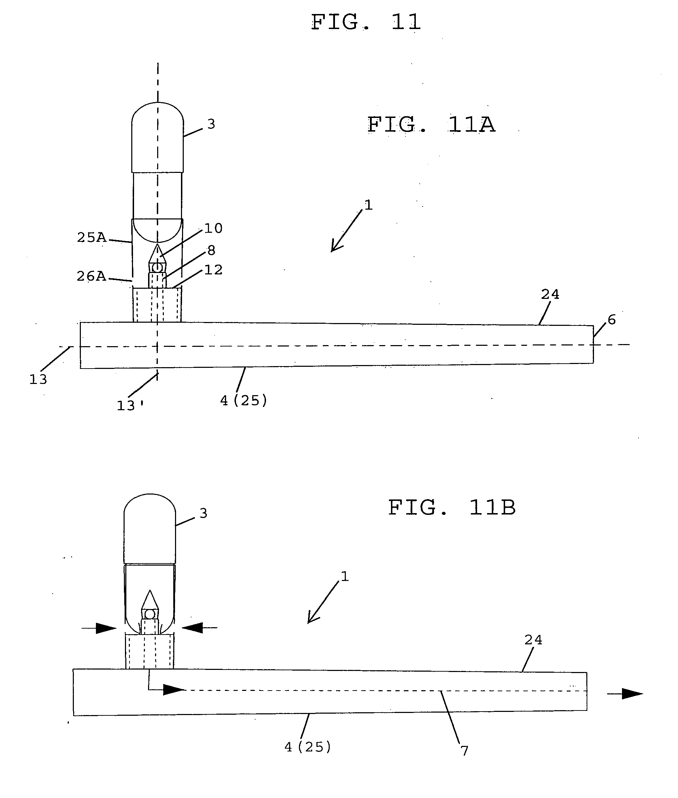

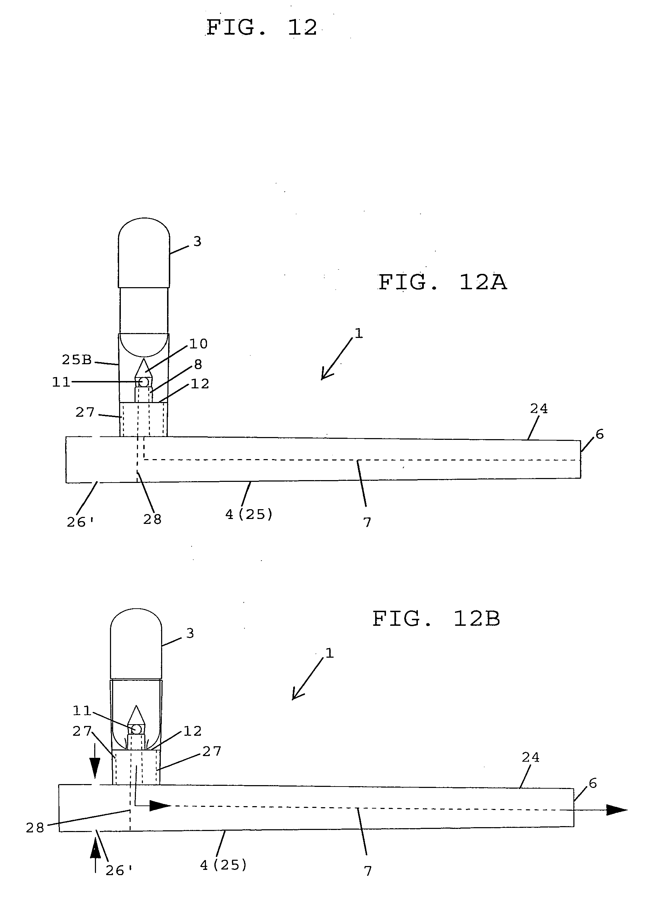

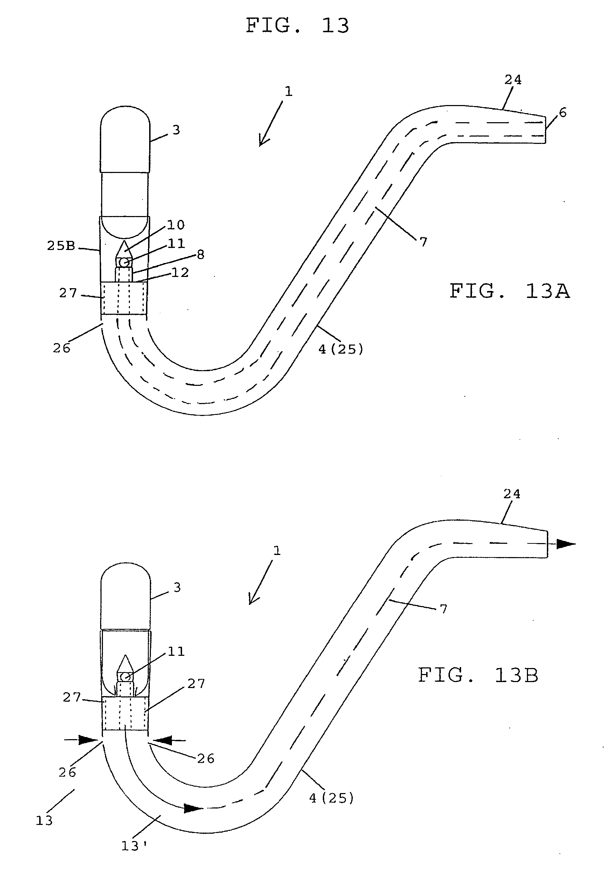

In Fig. 11 (und ähnlich in den Fig. 12 und 13) ist eine gegenüber den bisherigen Ausführungsformen insofern abgewandelte Vorrichtung 1 gezeigt, als insgesamt anstatt eines zigaretten- oder zigarenartigen Aussehens, mit koaxialer Anordnung eines geradlinigen Saugrohrkörpers und eines Schafts mit Spitze, eine pfeifenförmige Gestalt vorgesehen ist. Dabei ist in den Ausführungsformen gemäß Fig. 11A/11B sowie 12A/12B eine Ausbildung mit einem geraden Saugrohrkörper 4 (oder 25, wie aus Fig. 10A bis 10E oder Fig. 9A bis 9E ersichtlich) vorhanden, der geradlinig, ähnlich einem Zigarettenspitz, verläuft, der jedoch einen winkelig, etwa rechtwinkelig, vorgesehenen Anschluss für den Schaft 8, die Spitze 10 und damit die Kapsel 3 hat. Mit andren Worten, der Schaft 8, die Spitze 10 und die Kapsel 3 definieren eine Achse 13' (siehe Fig. 11A), die winkelig, insbesondere unter einem rechten Winkel, zur Längsachse 13 des Saugrohrkörpers verläuft. Entlang dieser Längsachse 13 des Saugrohrkörpers 4 verläuft auch der Saugkanal 7 im Saugrohrkörper, der in den Figuren 11B, 12A und 12B nur schematisch mit einer strichlierten Linie angedeutet ist.

Ähnlich wie bei der Ausführungsform gemäß Fig. 9A bis 9E ist bei den Ausführungsformen gemäß Fig. 11A, 11B und 12A, 12B ein allgemein scheibenförmiger Anschlagteil 12 für die Kapsel 3 vorgesehen, der jedoch seitlich am Saugrohrkörper 4 bzw. 25 angebracht ist, und an den der Schaft 8 mit der Spitze 10 anschließt. Die Ausbildung dieses Bereichs, wo die Kapsel 3 aufgesteckt wird, entspricht im Übrigen den bisher beschriebenen Ausführungsformen, so dass sich eine weitere Erläuterung erübrigen kann. Zu erwähnen ist nur noch im Zusammenhang mit der Ausführungsform gemäß Fig. 11A und 11B, dass hier ein Führungsteil 25A an den Anschlagteil 12 anschließt, wobei dieser Führungsteil 25A die Kapsel 3 beim Aufstechen führt, so dass die Kapsel 3 in der endgültigen Position gemäß Fig. 11B eine vorgegebene Stellung in Ausrichtung zur Achse 13' einnehmen kann, ähnlich wie vorstehend anhand der Fig. 9A bis 9E bzw. 10A bis 10E beschrieben wurde. Der Führungsteil 25A in der Ausführungsform gemäß Fig. 11A und 11B seitliche Lufteinlässe 26A, um so beim Ansaugen, vergleiche Fig. 11B, den Eintritt von Luft in den Bereich, wo die Kapsel 3 aufgestochen ist, zu ermöglichen. Insofern entspricht diese Ausführungsform in ihrer Funktion jener, wie sie bei den Ausführungsbeispielen gemäß Fig. 1 bis 8A/8B gegeben ist.

Im Gegensatz dazu weist die Ausführungsform gemäß Fig. 12A und 12B einen geschlossenen Führungsteil 25B für die Kapsel 3 beim Aufstechen auf die Spitze 10 auf. Ähnlich wie in der Ausführungsform gemäß Fig. 9A bis 9E bzw. 10A bis 10E wird hier über Luftkanäle 27 im Anschlagteil 12 Luft in den Bereich des aufgestochenen Kapselendes zugeführt, wobei diese Luft durch Lufteinlässe 26' im Saugrohrkörper 4 bzw. 25, und zwar vom Saugende 6 hergesehen jenseits des Anschlagteils 12, eintreten kann, vgl. insbesondere Fig. 12B mit den dort wiederum durch Pfeile veranschaulichten Luftströmungen.

Je nach Ausbildung des Saugkanals 7 im Saugrohrkörper 4 bzw. 25 kann, sollte ein einfacher Saugrohrkörper ähnlich jenem gemäß beispielsweise Fig. 5A und 5B gegeben sein, am Ende des Saugkanals 7 benachbart dem Anschluss zum Anschlagteil 12 eine in Fig. 12A und 12B nur ganz schematisch, mit strichlierter Linie, eingezeichnete Querwand vorhanden sein, um so einen Luftströmungs-Kurzschluss zu verhindern. In der Ausführungsform gemäß Fig. 11A und 11B wäre entsprechend der Saugrohrkörper 4 bzw. 25 am dem Saugende 6 gegenüberliegenden Ende abzuschließen.

In den Fig. 13A und 13B ist eine Vorrichtung 1 mit einem doppelt geschwungenen Saugrohrkörper 4 bzw. 25 gezeigt. Der Anschluss der Kapselaufnahme mit dem Schaft 8 und der Spitze 10 sowie dem Anschlagteil 12 ist dabei im Prinzip ähnlich wie in den Fig. 9A bis 9E gezeigt vorgesehen, wobei auch Lufteinlässe 26 und Luftkanäle 27 in entsprechender Weise vorgesehen sind, um im Bereich des aufgestochenen Kapselendes Luft zuzuführen und in das Kapselinnere eintreten zu lassen, wie dies vorstehend bereits erläutert wurde. Der Kapselführungsteil ist hier - ähnlich wie in Fig. 12A - mit 25B bezeichnet und entspricht hinsichtlich seiner Ausbildung und Funktion im Wesentlichen dem Führungsteil 25' gemäß Fig. 9A bis 9E.

In Abwandlung der doppelt geschwungenen Saugrohrkörperform gemäß Fig. 13A und 13B könnte auch der der Kapselaufnahme benachbarte Bogen weggelassen werden, so dass dort beispielsweise ein im Wesentlichen - in der Gebrauchslage - horizontales Saugrohrkörper-Stück vorliegt, an das dann die Kapselaufnahme mit dem Anschlagteil 12, dem Schaft 8 und der Spitze 10 sowie einem Führungsteil 25A bzw. 25B ähnlich wie in Fig. 11A/11B bzw. 12A/12B gezeigt angeschlossen sein kann.

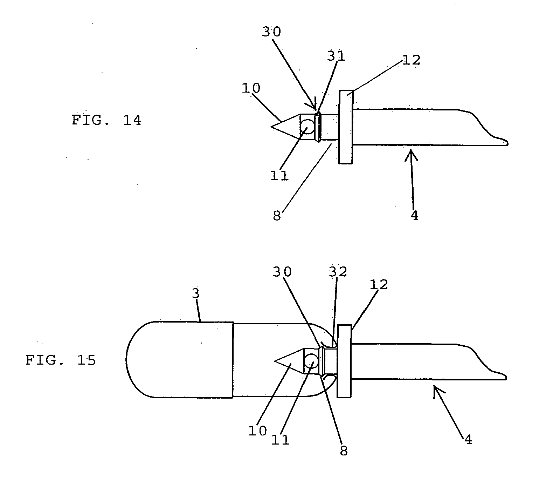

In Fig. 14 ist in einer Darstellung ähnlich Fig. 2 der Aufstechbereich eines Saugrohrkörpers 4 dargestellt, wobei wiederum der Schaft 8 mit dem Loch 11 sowie die Spitze 10 und weiters ein Anschlagteil 12 ersichtlich sind. Benachbart dem Loch 11 ist in der Ausführungsform gemäß Fig. 14 zusätzlich ein Rückhalte-Vorsprung 30 am Schaft 8 vorgesehen, insbesondere mit diesem einstückig geformt, wobei dieser Rückhalte-Vorsprung 30 im gezeigten Ausführungsbeispiel einfach durch einen Ringwulst 31 am Schaft 8 gebildet ist. Dieser Vorsprung 30 bzw. Ringwulst 31 zwischen dem Saugloch 11 und dem Anschlagteil 12 lässt einen an den Anschlagteil 12 anschließenden Abschnitt des Schafts 8 frei, wo, wie aus Fig. 15 ersichtlich ist, eine Kapsel 3 nach ihrem Aufstechen und Aufstecken auf die Spitze 10 und den Schaft 8 mit ihren Perforationsrand 32 zu liegen kommt. Der Perforationsrand 32 wird dabei zwischen dem Rückhalte-Vorsprung 30 und dem Anschlagteil 12 festgehalten, und der Rückhalte-Vorsprung 30 dient als Arretierungsmittel für die Kapsel 3, wobei er in der Art eines Widerhakens verhindert, dass die aufgesteckte Kapsel 3 ungewollt von der Spitze 10 bzw. vom Schaft 8 abrutschen kann.

Selbstverständlich kann auch ein sich nicht über den gesamten Umfang des Schaft 8 erstreckender Vorsprung 30 vorliegen, wie etwa ein aus diskontinuierlichen Bogenstücken über den Umfang des Schafts 8 verlaufender "geteilter" Vorsprung 30, um die gewünschte Arretierungsfunktion zu erfüllen.

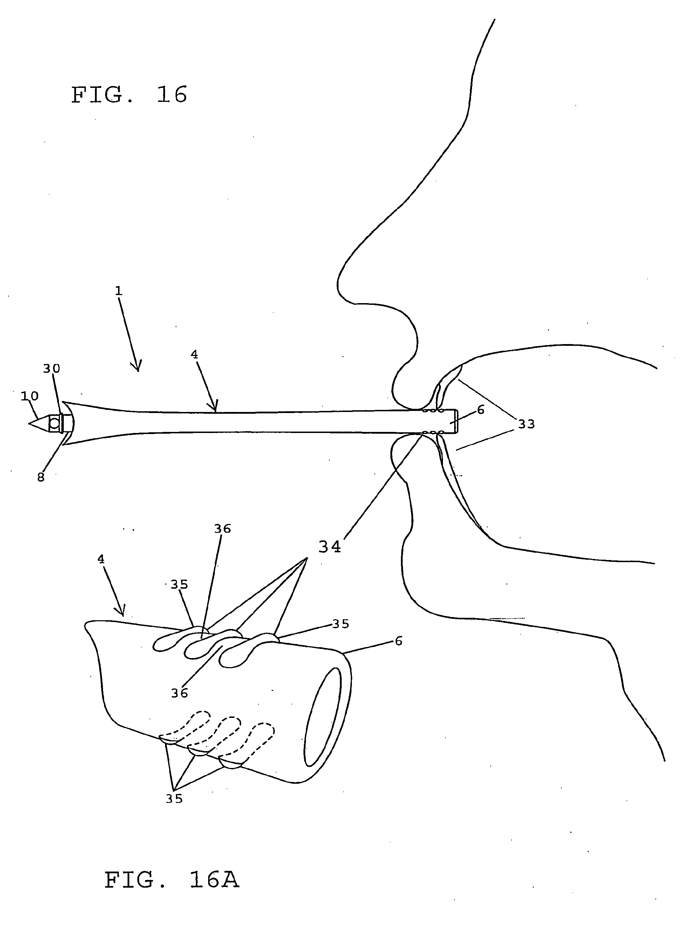

In Fig. 16 ist weiters eine Ausführungsform der vorliegenden Vorrichtung 1 ähnlich jener gemäß Fig. 8 gezeigt, die - abgesehen vom soeben beschriebenen Rückhalte-Vorsprung 30 am Aufstechende - mit ein Festhalten der Vorrichtung 1 zwischen den Schneidzähnen 33 eines Benützers begünstigenden Profilierungen 34 im Bereich des Saugendes 6 des Saugrohrkörpers 4 ausgebildet ist. Diese Profilierungen 34 können beispielsweise in Form von bogenförmigen Rippen 35 geformt sein, zwischen denen Vertiefungen 36 für die Schneidzähnen 33 vorliegen; anstelle dieser durch die Rippen 35 begrenzten Vertiefungen oder "Nuten" 36 können hohlkehlenartige Nuten im Saugrohrkörper 4 in vertiefter Anordnung vorgesehen sein, um in einer entsprechenden Weise das verbesserte Festhalten der Vorrichtung 1 mit den Zähnen zu ermöglichen.

Die Profilierungen 34, d.h. Rippen 35 bzw. Nuten 36, können sowohl wie gezeigt bogenförmig, nur über einen Teil des Umfangs des Saugrohrkörpers 4, vorzugsweise in einer aneinander gegenüberliegenden Ausrichtung, vorgesehen sein, als auch in einer kontinuierlichen Ringform.

Selbstverständlich sind, auch wenn vorstehend die erfindungsgemäße Vorrichtung 1 anhand besonders bevorzugter Ausführungsbeispiele erläutert wurde, weitere Abwandlungen und Modifikationen im Rahmen der Erfindung möglich. So kann etwa bei der Ausführungsform gemäß Fig. 10A bis 10E die Hülle 25 durchgehend zylindrisch sein, sie kann sich aber auch nur im letzten Abschnitt benachbart dem Saugende 6, verjüngen oder, je nachdem, gegebenenfalls auch erweitern, um ein Mundstück mit einem passenden Querschnitt zu erzielen. In der Ausführungsform gemäß Fig. 9A bis 9E kann der Saugrohrkörper 4 entsprechend jenem gemäß Fig. 8A und 8B ausgebildet sein. Bei allen Ausführungsformen ist es weiters denkbar, ein Ventil 16, wie anhand der Fig. 4 erläutert, wobei ein vergleichbares Saugventil auch im Inneren des Saugrohrkörpers 4 vorgesehen sein kann; und/oder einen Rückhalte-Vorsprung 30, wie anhand von Fig. 14 und 15 beschrieben; und/oder Festhalte-Profilierungen 34, wie anhand der Fig. 16 und 16A erläutert vorzusehen. Ferner ist es auch denkbar, in einer Mischform der Ausführungsbeispiele von Fig. 11A/11B (oder 12A/12B) und Fig. 13A/13B einen im Wesentlichen geradlinigen Saugrohrkörper 4 bzw. 25 vom Mundstück 24 weg vorzusehen, der an dem Saugende 6 gegenüberliegenden Ende über einen beispielsweise Viertelkreis-Bogen in eine nach oben gerichtete Kapselaufnahme entsprechend Fig. 13A, 13B übergeht. Allen Ausführungsformen gemeinsam ist jedenfalls das vorliegende Grundprinzip, dass bei Inbetriebnahme eine Kapsel nur einseitig geöffnet wird, und dass beim Ansaugen Luft zwischen dem aufgebrochenen Öffnungsrand der Kapsel und dem Schaft in das Kapselinnere eindringen kann, im Inneren der Kapsel verwirbelt wird und schließlich durch die Querlöcher und durch das Innere des Schafts sowie den Saugkanal angesaugt werden kann.

## Patentansprüche

1. Vorrichtung (1) zum Ansaugen von Pulver- oder Granulat-Material (2) aus einer Kapsel (3), mit einem Saugrohrkörper (4, 25), der einen Saugkanal (7) und ein Saugende (6) aufweist, und mit einer am Saugrohrkörper (4, 25) dem Saugende (6) gegenüberliegenden Kapsel-Aufnahme und einer Kapselaufstecheinrichtung (9), die eine zum nur einseitigen Aufstechen der Kapsel (3) eingerichtete Kapselaufstech-Spitze (10) aufweist, **dadurch gekennzeichnet, dass** die Kapsel-Aufnahme durch einen Schaft (8) gebildet ist, der mit dem Saugrohrkörper (4, 25) verbunden ist und die Kapselaufstech-Spitze (10) aufweist, wobei der Schaft (8) beim Aufstechen einer Kapsel (3) in deren Innerem zu liegen kommt, welcher Schaft (8) in seinem Inneren einen einzigen Saug-Längskanal (7') in direkter Verbindung mit dem Saugkanal (7) im Saugrohrkörper (4, 25) aufweist, welcher Saug-Längskanal (7') mit zumindest einem radial zur Längsachse des Schafts (8) verlaufenden, bei aufgesteckter Kapsel (3) in deren Innerem mündenden Saugloch (11) in Verbindung steht, wobei im Betrieb Luft zwischen dem Schaft (8) und dem Rand der Aufstech-Öffnung der aufgestochenen Kapsel (3) in deren Inneres einströmt und zusammen mit Pulver- oder Granulat-Material aus der Kapsel (3) durch das zumindest eine Saugloch (11), den einzigen Saug-Längskanal (7') im Schaft (8) sowie den Saugkanal (7) im Saugrohrkörper (4, 25) angesaugt wird.

2. Vorrichtung nach Anspruch 1, **dadurch gekennzeichnet, dass** sich der Schaft (8) in seinem der Spitze (10) benachbarten Teil (8B) von der Spitze (10) weg verjüngt.

3. Vorrichtung nach Anspruch 1 oder 2, **dadurch gekennzeichnet, dass** der Schaft (8) zumindest in seinem Saugrohrkörper-seitigen Teil (8A) zylindrisch ausgebildet ist.

4. Vorrichtung nach einer der Ansprüche 1 bis 3, **dadurch gekennzeichnet, dass** der Schaft (8) am von der Spitze (10) abgewandten Ende durch wenigstens einen Kapsel-Anschlagteil (12) begrenzt ist.

5. Vorrichtung nach Anspruch 4, **dadurch gekennzeichnet, dass** der Kapsel-Anschlagteil (12) eine, vorzugsweise zylindrisch gekrümmte Stirnfläche (15')aufweist.

6. Vorrichtung nach Anspruch 4, **dadurch gekennzeichnet, dass** der Kapsel-Anschlagteil (12) eine plane Stirnfläche (15) aufweist, wobei der Kapsel-Anschlagteil (12) vorzugsweise durch einen die plane Stirnfläche (15) aufweisenden Scheibenteil am Saugrohrkörper (4, 25) gebildet ist.

7. Vorrichtung nach einem der Ansprüche 4 bis 6, **dadurch gekennzeichnet, dass** sich der Saugrohrkörper (4) in seinem Querschnitt zum Kapsel-Anschlagteil (12) hin kontinuierlich auf dessen Umriss erweitert.

8. Vorrichtung nach einem der Ansprüche 1 bis 7, **dadurch gekennzeichnet, dass** der Saugrohrkörper (4) am Saugende (6) zu einem Mundstück (24) verdickt ist, und/oder dass sich der Saugkanal (7) zum Saugende (6) hin erweitert.

9. Vorrichtung nach einem der Ansprüche 1 bis 8, **dadurch gekennzeichnet, dass** dem Saugkanal (7) ein beim Ansaugen öffnendes Ventil (16) zugeordnet ist.

10. Vorrichtung nach einem der Ansprüche 1 bis 9, **dadurch gekennzeichnet, dass** dem Saugrohrkörper (4) eine über ihn aufschiebbare Hülse (25) zugeordnet ist.

11. Vorrichtung nach einem der Ansprüche 1 bis 10, **dadurch gekennzeichnet, dass** der Schaft (8) und die Spitze (10) koaxial zum Saugrohrkörper (4) angeordnet sind.

12. Vorrichtung nach einem der Ansprüche 1 bis 10, **dadurch gekennzeichnet, dass** der Schaft (8) und die Spitze (10) mit ihrer Achse (13') winkelig, z.B. rechtwinkelig, zum Saugrohrkörper (4; 25) angeordnet ist, und/oder dass der Saugrohrkörper (4; 25) einen in der Art einer Pfeife geschwungenen Verlauf hat.

13. Vorrichtung nach einem der Ansprüche 1 bis 12, **dadurch gekennzeichnet, dass** der Schaft (8) auf der dem Saugende (6) näherliegenden Seite des Lochs (11) wenigstens einen Rückhalte-Vorsprung (30), z.B. in Form eines ringförmigen Wulsts (31), für eine aufgesteckte Kapsel (3) aufweist.

14. Vorrichtung nach einem der Ansprüche 1 bis 13, **dadurch gekennzeichnet, dass** der Saugrohrkörper (4) im Bereich des Saugendes (6) wenigstens eine Profilierung (34), z.B. Profilierungen in Form von Rippen (35) oder Nuten (36) aufweist, um das Festhalten des Saugrohrkörpers (4) mit den Lippen bzw. Zähnen zu erleichtern.

15. Vorrichtung (1) nach einem der Ansprüche 1 bis 14, mit einer Kapsel (3) mit einem allgemein zylindrischen Kapselkörper, der an den Enden auswärts gewölbte Stirnflächen (22, 23) aufweist, **dadurch gekennzeichnet, dass** an zumindest einer auswärts gewölbten Stirnfläche (22, 23) mittig eine, ein Perforieren mittels der Spitze (10) erleichternde Führungs- oder Schwächungsstelle (18) vorgesehen ist, die durch eine dellenförmige Vertiefung (19) oder eine Materialverdünnung (18') der Kapselwand gebildet ist.

## Claims

1. An apparatus (1) for taking in powder or granule material (2) from a capsule (3), comprising a suction tube body (4, 25) which comprises a suction channel (7) and a suction end (6), and a capsule holder disposed at the suction tube body (4, 25) opposite of the suction end (6), and a capsule piercing device (9) comprising a capsule piercing tip (10) designed for piercing the capsule (3) on only one side, **characterized in that** the capsule holder is formed by a shaft (8) that is connected with the suction tube body (4, 25) and comprises the capsule piercing tip (10), wherein the shaft (8) during the piercing of a capsule (3) is positioned within the interior thereof, said shaft (8) comprising in the interior thereof a single suction longitudinal channel (7') in direct communication with the suction channel (7) in the suction tube body (4, 25), said suction longitudinal channel (7') being in communication with at least one suction hole (11) extending radially to the longitudinal axis of the shaft (8) and opening into the interior of the capsule when the capsule (3) is attached, wherein in operation air between the shaft (8) and the edge of the pierced opening of the pierced capsule (3) flows into the interior thereof and is sucked along with powder or granule material, from the capsule (3) through the at least one suction hole (11), the only suction longitudinal channel (7') in the shaft (8), and the suction channel (7) in the suction tube body (4, 25).

2. The apparatus according to claim 1, **characterized in that** the shaft (8) tapers in the portion (8B) adjacent to the tip (10) in a direction away from the tip (10).

3. The apparatus according to claims 1 or 2, **characterized in that** the shaft (8) is designed cylindrically at least in the suction tube body-side portion (8A) thereof.

4. The apparatus according to any of claims 1 to 3, **characterized in that** the shaft (8) is confined by at least one capsule abutment element (12) at the end facing away from the tip (10).

5. The apparatus according to claim 4, **characterized in that** the capsule abutment element (12) comprises a front face (15') that is preferably curved cylindrically.

6. The apparatus according to claim 4, **characterized in that** the capsule abutment element (12) comprises a plane front face (15), wherein the capsule abutment element (12) is preferably formed by a disc portion at the suction tube body (4, 25) which comprises the plane front face (15).

7. The apparatus according to any of claims 4 to 6, **characterized in that** the cross-section of the suction tube body (4) continuously widens toward the capsule abutment element (12) until it has reached the contour thereof.

8. The apparatus according to any of claims 1 to 7, **characterized in that** the suction tube body (4) is thickened to form a mouth piece (24) at the suction end (6), and/or that the suction channel (7) widens toward the suction end (6).

9. The apparatus according to any of claims 1 to 8, **characterized in that** a valve (16) opening during intake is associated with the suction channel (7).

10. The apparatus according to any of claims 1 to 9, **characterized in that** the suction tube body (4) has associated therewith a shell (25) adapted to be slid thereon.

11. The apparatus according to any of claims 1 to 10, **characterized in that** the shaft (8) and the tip (10) are arranged coaxially to the suction tube body (4).

12. The apparatus according to any of claims 1 to 10, **characterized in that** the shaft (8) and the tip (10) with their axes (13') are arranged at an angle, e.g. at a right angle, to the suction tube body (4; 25), and/or that the suction tube body (4; 25) has a progression curved like a pipe.

13. The apparatus according to any of claims 1 to 12, **characterized in that** the shaft (8) comprises, at the side of the hole (11) which is closer to the suction end (6), at least one retention projection (30), e.g. in the form of an annular bead (31), for an attached capsule.

14. The apparatus according to any of claims 1 to 13, **characterized in that** the suction tube body (4) comprises, in the region of the suction end (6), at least one profiling (34), e.g. profilings in the form of ribs (35) or grooves (36), so as to facilitate the retaining of the suction tube body (4) with the lips and/or teeth.

15. The apparatus (1) according to any of claims 1 to 14, comprising a generally cylindrical capsule body having outwardly curved front faces (22, 23) at the ends thereof, **characterized in that** a guiding or weakening point (18) is provided in the middle of at least one outwardly curved front face (22, 23) which facilitates a perforation by means of the tip (10) and which is formed by a dent-shaped recess (19) or a material thinning (18') of the capsule wall.

## Revendications

1. Dispositif (1) pour aspirer un matériau (2) en poudre ou en granulés à partir d'une gélule (3), comprenant un corps tubulaire d'aspiration (4, 25), qui présente un canal d'aspiration (7) et une extrémité d'aspiration (6), et comprenant également un système d'accueil de gélule opposé à l'extrémité d'aspiration (6) sur le corps tubulaire d'aspiration (4, 25), ainsi qu'un système de transpercement de gélule (9), qui présente une pointe de transpercement de gélule (10) conçue pour transpercer la gélule (3) uniquement sur un seul côté, **caractérisé en ce que** le système d'accueil de gélule est formé par un embout allongé (8), qui est relié au corps tubulaire d'aspiration (4, 25) et présente la pointe de transpercement de gélule (10), l'embout allongé (8) venant, lors du transpercement de la gélule (3), se positionner à l'intérieur de cette dernière, et l'embout allongé (8) présentant, en son intérieur, un seul canal longitudinal d'aspiration (7'), qui est en liaison directe avec le canal d'aspiration (7) dans le corps tubulaire d'aspiration (4, 25), ce canal longitudinal d'aspiration (7') étant en liaison avec au moins un trou d'aspiration (11), qui s'étend radialement par rapport à l'axe longitudinal de l'embout allongé (8), et débouche, lorsque la gélule (3) est emmanchée, à l'intérieur de celle-ci, et **en ce qu'**en service, de l'air s'écoule entre l'embout allongé (8) et le bord de l'ouverture de transpercement de la gélule (3) transpercée, vers l'intérieur de cette dernière, et est aspiré, en commun avec du matériau en poudre ou en granulés, à partir de la gélule (3), à travers ledit au moins un trou d'aspiration (11), l'unique canal longitudinal d'aspiration (7') dans l'embout allongé (8), ainsi que le canal d'aspiration (7) dans le corps tubulaire d'aspiration (4, 25).

2. Dispositif selon la revendication 1, **caractérisé en ce que** l'embout allongé (8) se rétrécit en s'éloignant de la pointe (10), dans sa partie (8B) voisine de la pointe (10).

3. Dispositif selon la revendication 1 ou la revendication 2, **caractérisé en ce que** l'embout allongé (8) est de configuration cylindrique au moins dans sa partie (8A) située du côté du corps tubulaire d'aspiration.

4. Dispositif selon l'une des revendications 1 à 3, **caractérisé en ce que** l'embout allongé (8) est limité, à son extrémité éloignée de la pointe (10), par au moins une partie de butée de gélule (12).

5. Dispositif selon la revendication 4, **caractérisé en ce que** la partie de butée de gélule (12) présente une surface frontale (15'), de préférence à courbure cylindrique.

6. Dispositif selon la revendication 4, **caractérisé en ce que** la partie de butée de gélule (12) présente une surface frontale plane (15), la partie de butée de gélule (12) étant de préférence formée par une partie en forme de disque, qui présente la surface frontale plane (15), sur le corps tubulaire d'aspiration (4, 25).

7. Dispositif selon l'une des revendications 4 à 6, **caractérisé en ce que** le corps tubulaire d'aspiration (4) s'évase, dans sa section transversale, de manière continue en direction de la partie de butée de gélule (12), jusqu'à la périphérie de celle-ci.

8. Dispositif selon l'une des revendications 1 à 7, **caractérisé en ce que** le corps tubulaire d'aspiration (4), est renflé à l'extrémité d'aspiration (6), en une pièce buccale (24), et/ou **en ce que** le canal d'aspiration (7) s'évase en direction de l'extrémité d'aspiration (6).

9. Dispositif selon l'une des revendications 1 à 8, **caractérisé en ce qu'**au canal d'aspiration (7) est associée une valve (16) ouvrant lors de l'aspiration.

10. Dispositif selon l'une des revendications 1 à 9, **caractérisé en ce qu'**au corps tubulaire d'aspiration (4) est associé un fourreau (25) pouvant être emmanché pardessus.

11. Dispositif selon l'une des revendications 1 à 10, **caractérisé en ce que** l'embout allongé (8) et la pointe (10) sont agencés coaxialement au corps tubulaire d'aspiration (4).

12. Dispositif selon l'une des revendications 1 à 10, **caractérisé en ce que** l'embout allongé (8) et la pointe (10) sont agencés, avec leur axe (13'), de manière angulaire, par exemple à angle droit, par rapport au corps tubulaire d'aspiration (4; 25), et/ou **en ce que** le corps tubulaire d'aspiration (4; 25) présente une allure incurvée à la manière d'une pipe.

13. Dispositif selon l'une des revendications 1 à 12, **caractérisé en ce que** l'embout allongé (8) présente, sur le côté du trou (11), qui est le plus proche de l'extrémité d'aspiration (6), au moins une protubérance de retenue (30), par exemple sous la forme d'un bourrelet annulaire (31), pour une gélule (3).

14. Dispositif selon l'une des revendications 1 à 13, **caractérisé en ce que** le corps tubulaire d'aspiration (4) présente, dans la zone de l'extrémité d'aspiration (6), au moins un profilage (34), par exemple des profilages sous la forme de nervures (35) ou de rainures (36), en vue de faciliter la tenue du corps tubulaire d'aspiration (4) à l'aide des lèvres ou des dents.

15. Dispositif selon l'une des revendications 1 à 14, avec une gélule (3) à corps de gélule cylindrique de manière générale, qui présente, à ses extrémités, des surfaces frontales (22, 23) bombées vers l'extérieur, **caractérisé en ce que** sur au moins une surface frontale (22, 23) bombée vers l'extérieur, est prévue, au centre, une zone de guidage ou d'affaiblissement (18) facilitant une perforation au moyen de la pointe (10), et qui est formée par un creux (19) en forme de petite bosse, ou par un amincissement de matière (18') de la paroi de gélule.
